# EUROPEAN PATENT APPLICATION

(11) **EP 3 305 229 A1**
(43) Date of publication of application: **11.04.2018**
(21) Application number: 15894281.3
(22) Date of filing: 31.08.2015
(51) Int. Cl.: A61B 18/00

(54) **MEDICAL MANIPULATOR**

(30) Priority: 01.06.2015 US 201562168987 P
(71) Applicant: Olympus Corporation, Hachioji-shi Tokyo 192-8507 (JP)
(72) Inventor: HYODO, Ryoji, Hachioji-shi Tokyo 192-8507 (JP); TAKEMOTO, Shotaro, Hochioji-shi Tokyo 192-8507 (JP)
(74) Representative: Schicker, Silvia
(86) International application number: PCT/JP2015/074792
(87) International publication number: WO 2016/194249

(57) **Abstract**

A medical manipulator (1) according to the present invention includes: a flexible elongated section (2) composed of an electrically conductive material and capable of transmitting torsional torque; a joint section (3) disposed at a distal end of the elongated section (2), having a joint (14, 15, 16), and composed of an electrically conductive material; a treatment section (4) attached to a distal end of the joint section (3); a cylindrical connection member (6) composed of an insulating material, disposed between the elongated section (2) and the joint section (3), and connecting the elongated section (2) and the joint section (3); an electric cable that extends internally through the elongated section (2), the connection member (6), and the joint section (3) in a state where the electric cable is electrically insulated from the elongated section (2) and the joint section (3); and a cover composed of an insulating material and covers outer surfaces of the joint section (3) and the elongated section (2).

## Description

### {Technical Field}

The present invention relates to medical manipulators.

### {Background Art}

In a known medical manipulator having a treatment section, such as an electric scalpel, at the distal end of an elongated section with a metallic joint section interposed therebetween, a metallic portion of, for example, the joint section near the treatment section is covered with a cover composed of an insulating material so as to prevent the metallic portion from coming into contact with surrounding tissue (for example, see PTL 1 and PTL 2).

Furthermore, in a medical manipulator having a treatment section at the distal end of a flexible elongated section with a joint section interposed therebetween, a metallic coil tube is normally used to provide the elongated section with flexibility, torque transmissibility, and compressibility.

An electric cable that supplies an AC voltage to the treatment section is routed internally through the joint section and the elongated section, and the metallic joint section and the metallic coil tube are insulated from each other by a sheath or tube composed of an insulating material.

As mentioned above, the joint section and the coil tube are connected to each other for transmitting torque, and a metallic wire for driving the joint section is connected to the joint section.

### {Citation List}

### {Patent Literature}

{PTL 1}
   Japanese Unexamined Patent Application, Publication No. 2012-70953
{PTL 2}
   Japanese Unexamined Patent Application, Publication No. 2012-152562

### {Summary of Invention}

### {Technical Problem}

For the reasons above, capacitor elements are formed between the electric cable and the elements including the joint section, the wire, and the coil tube. Some coil tubes are long up to 2 meters or more, causing the capacitance of a capacitor element to become extremely large. When an AC voltage is supplied to the electric cable for medically treating tissue, if the cover that covers the joint section near the treatment section comes into contact with the tissue, the cover may undergo dielectric breakdown due to a large electric current flowing therethrough.

Furthermore, with regard to the cover that covers the joint section near the treatment section, it is difficult to increase the wall thickness due to the need to reduce the outer diameter of the joint section without inhibiting the movement of the joint section, thus making it difficult to improve the insulation performance of the cover.

The present invention has been made in view of the circumstances described above, and an object thereof is to provide a medical manipulator that can prevent dielectric breakdown of a cover, which covers a joint section near a treatment section, without increasing the wall thickness of the cover so as to prevent electric current from flowing to sections other than the treatment section.

### {Solution to Problem}

An aspect of the present invention provides a medical manipulator including: a flexible elongated section that includes a component composed of an electrically conductive material and that is capable of transmitting torsional torque; a joint section that is disposed at a distal end of the elongated section and that has at least one joint and includes a component composed of an electrically conductive material; a treatment section that is attached to a distal end of the joint section and that medically treats tissue; a cylindrical connection member that is composed of an insulating material and that is disposed between the elongated section and the joint section to connect the elongated section and the joint section; an electric cable that extends internally through the elongated section, the connection member, and the joint section in a state where the electric cable is electrically insulated from the elongated section and the joint section and that supplies electric power to the treatment section; and a cover that is composed of an insulating material and that covers outer surfaces of the joint section and the elongated section.

According to this aspect, the medical manipulator is inserted into the body of a patient through a channel of, for example, an endoscope or an overtube inserted in the body of the patient, from the treatment section. Then, the treatment section is brought closer to the treatment site, the position and orientation of the treatment section are adjusted by driving the joint section, and electric power is supplied to the electric cable, whereby the treatment site is appropriately treated by using the treatment section.

In this case, since the connection member that connects the joint section and the elongated section is composed of an insulating material, the capacitance of a capacitor element formed between the joint section and the electric cable can be well reduced to a small value. As a result, even if the joint section is driven and comes into contact with tissue near the treatment site, dielectric breakdown of the cover does not occur, so that the wall thickness of the cover can be reduced, thereby achieving a reduced diameter of the joint section. Moreover, by reducing the thickness of the cover, the movement of the joint section can be prevented from being inhibited by the cover.

In the above aspect, the connection member may connect the elongated section and the joint section such that torsional torque can be transmitted between the elongated section and the joint section.

Accordingly, in a case where the orientation of the treatment section is to be rotated about the longitudinal axis of the elongated section, torsional torque is applied at the proximal end of the elongated section so that the torsional torque transmitted by the elongated section is transmitted to the joint section via the connection member, whereby the orientation of the treatment section can be changed.

In the above aspect, the joint section and the connection member may individually have joint-side protrusion-recess sections that are fitted to each other in a detachable manner in a direction extending along an axis of the connection member and that engage with each other in a fitted state so that the joint section and the connection member do not relatively move in a circumferential direction.

Accordingly, by simply fitting the joint section and the connection member to each other in the direction extending along the axis of the connection member, the joint-side protrusion-recess sections provided in the joint section and the connection member can be engaged with each other, whereby torsional torque can be reliably transmitted between the connection member and the joint section.

Furthermore, in the above aspect, the connection member and the elongated section may individually have elongated-section-side protrusion-recess sections that are fitted to each other in a detachable manner in a direction extending along an axis of the connection member and that engage with each other in a fitted state so that the connection member and the elongated section do not relatively move in a circumferential direction.

Accordingly, by simply fitting the elongated section and the connection member to each other in the direction extending along the axis of the connection member, the elongated-section-side protrusion-recess sections provided in the elongated section and the connection member can be engaged with each other, whereby torsional torque can be reliably transmitted between the elongated section and the connection member.

Furthermore, in the above aspect, the cover may include a joint cover that covers the joint section and an elongated section cover that covers the elongated section, and the joint cover and the elongated-section cover may extend to a position where the covers partially cover the connection member.

Accordingly, the joint cover covers the joint section, the elongated-section cover covers the elongated section, and the joint cover and the elongated-section cover extend to positions where they partially cover the connection member composed of an insulating material, so that the part composed of an electrically conductive material is completely covered and is not exposed to the outside.

### {Advantageous Effects of Invention}

The present invention is advantageous in that dielectric breakdown of a cover, which covers a joint section near a treatment section, is prevented without increasing the wall thickness of the cover, so that electric current can be prevented from flowing to sections other than the treatment section.

### {Brief Description of Drawings}

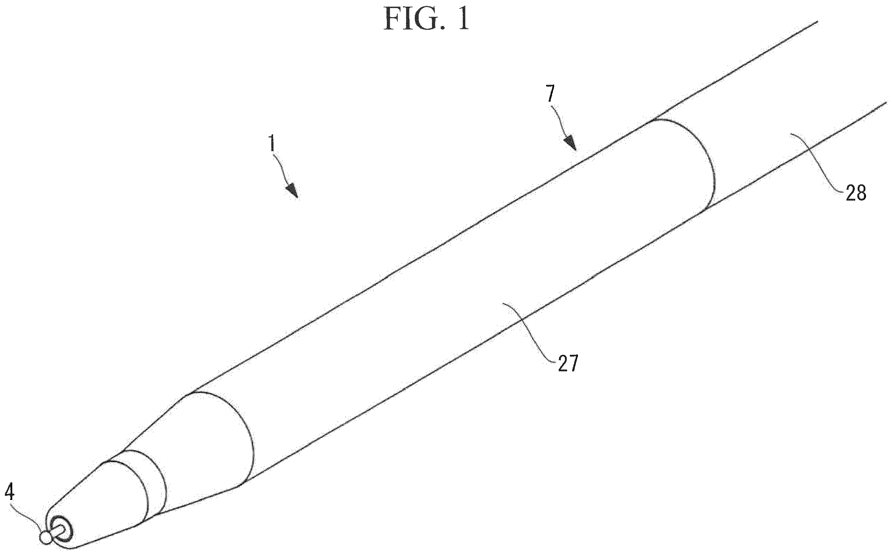

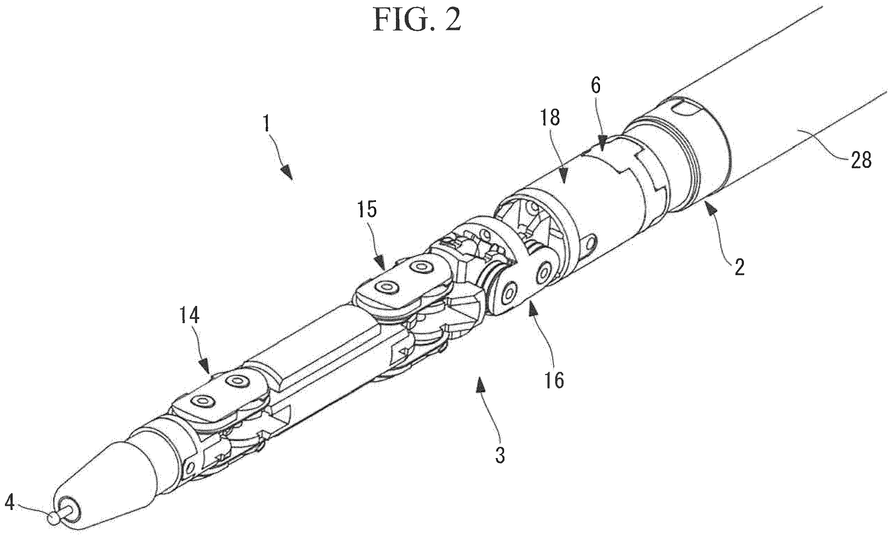

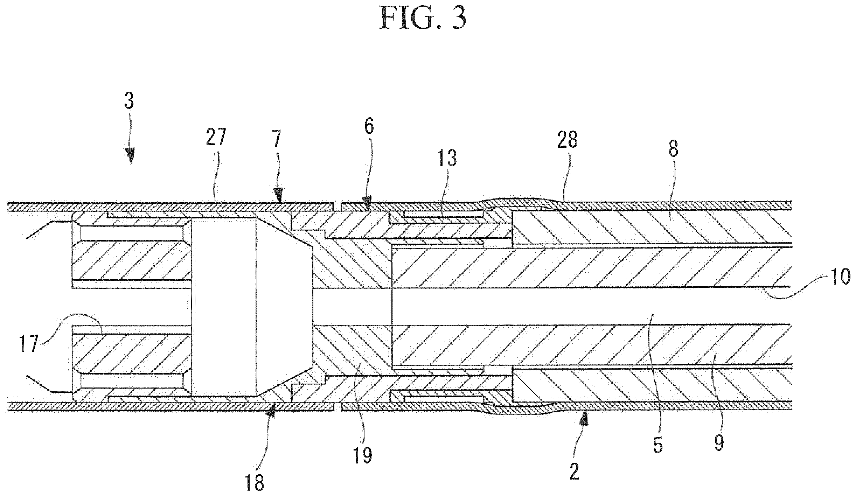

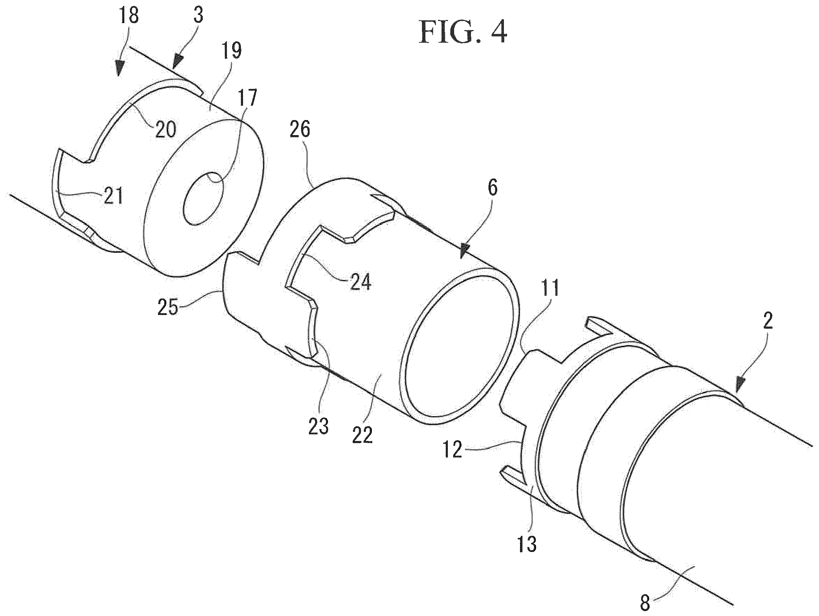

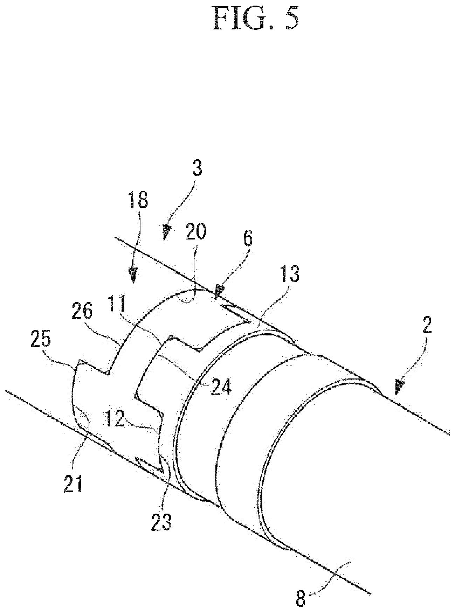

{Fig. 1} Fig. 1 is a partial perspective view illustrating a medical manipulator according to an embodiment of the present invention.
{Fig. 2} Fig. 2 is a partial perspective view illustrating a state where a joint cover of the medical manipulator in Fig. 1 is removed.
{Fig. 3} Fig. 3 is a partial longitudinal sectional view illustrating a connected state of a joint section, a connection member, and an elongated section of the medical manipulator in Fig. 1.
{Fig. 4} Fig. 4 is a partial perspective view illustrating a disconnected state of the joint section, the connection member, and the elongated section of the medical manipulator in Fig. 1.
{Fig. 5} Fig. 5 is a partial perspective view illustrating a combined state of the joint section, the connection member, and the elongated section in Fig. 4.

### {Description of Embodiments}

A medical manipulator 1 according to an embodiment of the present invention will be described below with reference to the drawings.

As shown in Figs. 1 and 2, the medical manipulator 1 according to this embodiment is to be inserted into the body of a patient through a channel provided in a flexible insertion section of, for example, an endoscope or an overtube and includes a flexible elongated section 2 that is bendable in conformity to a winding channel, a joint section 3 attached to the distal end of the elongated section 2, a treatment section 4 supported by the distal end of the joint section 3, an electric cable 5 for supplying electric power to the treatment section 4, a connection member 6 that connects the elongated section 2 and the joint section 3, and a cover 7 that covers the joint section 3 and the elongated section 2.

As shown in Fig. 3, the elongated section 2 includes a coil tube 8, formed by winding a metallic wire rod into a shape of a tightly wound coil, and a multi-lumen tube 9 extending internally through the coil tube 8.

The multi-lumen tube 9 is composed of a flexible resin material, such as silicone resin, and has a plurality of through-holes 10 through which, for example, wires (not shown) for driving the electric cable 5 and the joint section 3 extend.

As shown in Fig. 4, a tubular protrusion-recess member 13 having a plurality of (four in the example in the drawing) protrusions (elongated-section-side protrusion-recess section) 11 and a plurality of recesses (elongated-section-side protrusion-recess section) 12 alternately arranged in the circumferential direction is fixed to the distal end of the coil tube 8.

The joint section 3 includes three joints 14, 15, and 16 that tilt about axes orthogonal to the longitudinal axis of the elongated section 2 and is formed by combining a plurality of metallic components.

The inside of the joint section 3 is provided with a through-hole 17 through which the electric cable 5 extends so that electric power can be supplied to the treatment section 4 supported at the distal end thereof.

Furthermore, as shown in Fig. 4, a base member 18 disposed at the proximal end of the joint section 3 includes a cylindrical fitting section 19, and the outer peripheral surface of the fitting section 19 is provided with a plurality of (two in the example in the drawing) protrusions (joint-side protrusion-recess section) 20 and a plurality of recesses (joint-side protrusion-recess section) 21 extending in the axial direction and alternately arranged in the circumferential direction, similarly to the protrusion-recess member 13 attached to the coil tube 8.

The treatment section 4 is, for example, an electric scalpel having an electrode at the distal end thereof.

The electric cable 5 is formed by covering a metallic electric wire with a sheath composed of an insulating material.

As shown in Fig. 4, the connection member 6 is a cylindrical member and includes a fitting section 22 that fits into the protrusion-recess member 13 provided at the distal end of the aforementioned coil tube 8. Moreover, as shown in Fig. 5, the connection member 6 includes protrusions (elongated-section-side protrusion-recess section) 23 to be combined with the recesses 12 of the protrusion-recess member 13 and recesses (elongated-section-side protrusion-recess section) 24 to be combined with the protrusions 11 of the protrusion-recess member 13 when the fitting section 22 is fitted onto the inside surface of the protrusion-recess member 13, and the protrusions 23 and the recesses 24 are arranged in the circumferential direction. The shape of the protrusions and recesses at the proximal end of the connection member 6 is substantially complementary to the shape of the protrusions and recesses of the protrusion-recess member 13.

The distal end of the connection member 6 is provided with protrusions (joint-side protrusion-recess section) 25 to be combined with the recesses 21 of the base member 18 and recesses (joint-side protrusion-recess section) 26 to be combined with the protrusions 20 of the base member 18 when the fitting section 19 of the base member 18 of the joint section 3 is fitted with the connection member 6, and the protrusions 25 and the recesses 26 are arranged in the circumferential direction. The shape of the protrusions and recesses at the distal end of the connection member 6 is also substantially complementary to the shape of the protrusions and recesses of the base member 18.

The connection member 6 is composed of a rigid insulating material, such as PEEK, ceramic, or polyethylene.

When the base member 18 of the joint section 3, the connection member 6, and the protrusion-recess member 13 of the coil tube 8 are fitted to one another, the recesses 12, 21, 24, 26 and the protrusions 11, 20, 23, and 25 are engaged and connected with each other, as shown in Fig. 5, so as not to move relative to one another in the circumferential direction.

Accordingly, torsional torque transmitted by the coil tube 8 is transmitted to the connection member 6 by the engagement between the recesses 12 and 24 and the protrusions 11 and 23, and is also transmitted to the base member 18.

Since a wire for driving the joint section 3 is disposed within the joint section 3 and the elongated section 2 and a tensile force is applied to the wire for driving the joint section 3, the base member 18 of the joint section 3, the connection member 6, and the protrusion-recess member 13 of the coil tube 8 do not move away from one another in the axial direction even if they are not locked to one another in the axial direction, whereby the fitted state is maintained.

As shown in Fig. 1, the cover 7 includes a joint cover 27 that covers the outer surface of the joint section 3 and an elongated-section cover 28 that covers the outer surface of the elongated section 2.

As shown in Fig. 3, the joint cover 27 and the elongated-section cover 28 extend to positions where each of them partially covers the connection member 6 and they are disposed such that the ends thereof face each other.

The joint cover 27 is composed of silicone resin, fluororesin, or urethane resin and has a wall thickness of, for example, 0.2 mm.

The elongated-section cover 28 is composed of urethane resin, FEP, or silicone resin and has a wall thickness of, for example, 0.2 mm.

The operation of the medical manipulator 1 according to this embodiment having the above-described configuration will be described below.

In order to medically treat a treatment site inside the body of a patient by using the medical manipulator 1 according to this embodiment, the medical manipulator 1 is inserted through a channel provided in, for example, an endoscope or an overtube inserted in the body of the patient, starting from the treatment section 4. Then, the treatment section 4 protruding from the distal end of the channel is brought closer to the treatment site.

Subsequently, the position and orientation of the treatment section 4 are adjusted by driving the joint section 3. Moreover, by applying torsional torque about the longitudinal axis of the elongated section 2 at the proximal end of the elongated section 2, the joint section 3 is rotated by the torsional torque transmitted by the elongated section 2, whereby the orientation of the treatment section 4 about the longitudinal axis of the elongated section 2 can be adjusted.

Specifically, because the elongated section 2 is formed of the coil tube 8, torsional torque applied to the proximal end can be reliably transmitted to the distal end. Furthermore, the protrusion-recess member 13 fixed to the distal end of the coil tube 8 is combined with the connection member 6 and is engaged therewith in the circumferential direction. Moreover, the connection member 6 and the base member 18 are also combined with each other and are engaged with each other in the circumferential direction, so that the torsional torque transmitted by the coil tube 8 is reliably transmitted to the joint section 3 via the connection member 6 and the base member 18.

In the medical manipulator 1 according to this embodiment, the electric cable 5 is routed by means of the multi-lumen tube 9 and the insulating material covering the electric cable 5, which extend internally through the coil tube 8 and the joint section 3 along the entire length thereof, so that capacitor elements are formed between the coil tube 8 and the electric cable 5 and between the joint section 3 and the electric cable 5 when alternating current is applied.

In this case, since the connection member 6 composed of an insulating material is disposed between the coil tube 8 and the joint section 3, exchanging of electric charge is not performed between the elongated section 2 and the joint section 3. Therefore, the capacitor element in the joint section 3 is small and independent from the capacitor element in the elongated section 2, and the capacitance in this case is reduced to a sufficiently small value. As a result, even when the joint section 3 is moved to come into contact with tissue surrounding the treatment site, large electric charge is not accumulated since the capacitor element is minimized. This is advantageous in that leakage current can be sufficiently and effectively reduced or blocked by the joint cover 27, which is extremely thin.

Specifically, since the joint cover 27 is thin, the outer diameter of the joint section 3 can be sufficiently reduced. Accordingly, this enables improved ease of insertion through the channel. Moreover, by reducing the thickness of the joint cover 27, the movement of the joint section 3 is prevented from being inhibited by the cover 7, which is advantageous in terms of improved ease of operation of the joint section 3.

As an alternative to this embodiment in which the joint section 3 is constituted of metallic components, the joint section 3 may include components composed of an electrically conductive material and may be constituted of a torsional-torque transmittable member, such as a resinous component.

As an alternative to this embodiment in which torsional torque is transmitted from the elongated section 2 by the connection member 6 that insulates the elongated section 2 and the joint section 3 from each other, the medical manipulator 1 may include an operating section (not shown) to which torsional torque is input, and the joint section 3 may generate torsional torque on the basis of the input from the operating section. In this case, the operating section is, for example, a wire. By pushing and pulling the wire, torque may be generated in the joint section 3 about the longitudinal axis of the elongated section 2.

### {Reference Signs List}

1 medical manipulator
2 elongated section
3 joint section
4 treatment section
5 electric cable
6 connection member
11, 20, 23, 25 protrusion (joint-side protrusion-recess section, elongated-section-side protrusion-recess section)
12, 21, 24, 26 recess (joint-side protrusion-recess section, elongated-section-side protrusion-recess section)
14, 15, 16 joint
27 joint cover
28 elongated-section cover

## Claims

1. A medical manipulator comprising:
a flexible elongated section that includes a component composed of an electrically conductive material and that is capable of transmitting torsional torque;
a joint section that is disposed at a distal end of the elongated section and that has at least one joint and includes a component composed of an electrically conductive material;
a treatment section that is attached to a distal end of the joint section and that medically treats tissue;
a cylindrical connection member that is composed of an insulating material and that is disposed between the elongated section and the joint section to connect the elongated section and the joint section;
an electric cable that extends internally through the elongated section, the connection member, and the joint section in a state where the electric cable is electrically insulated from the elongated section and the joint section and that supplies electric power to the treatment section; and
a cover that is composed of an insulating material and that covers outer surfaces of the joint section and the elongated section.

2. The medical manipulator according to Claim 1,
wherein the connection member connects the elongated section and the joint section such that torsional torque can be transmitted between the elongated section and the joint section.

3. The medical manipulator according to Claim 1 or 2,
wherein the joint section and the connection member individually have joint-side protrusion-recess sections that are fitted to each other in a detachable manner in a direction extending along an axis of the connection member and that engage with each other in a fitted state so that the joint section and the connection member do not relatively move in a circumferential direction.

4. The medical manipulator according to any one of Claims 1 to 3,
wherein the connection member and the elongated section individually have elongated-section-side protrusion-recess sections that are fitted to each other in a detachable manner in a direction extending along an axis of the connection member and that engage with each other in a fitted state so that the connection member and the elongated section do not relatively move in a circumferential direction.

5. The medical manipulator according to any one of Claims 1 to 4,
wherein the cover includes a joint cover that covers the joint section and an elongated section cover that covers the elongated section, and
wherein the joint cover and the long-section cover extend to a position where the covers partially cover the connection member.
